# EUROPÄISCHE PATENTSCHRIFT

(11) **EP 1 959 882 B1**
(45) Veröffentlichungstag und Bekanntmachung des Hinweises auf die Patenterteilung: **12.02.2014**
(21) Anmeldenummer: 06828592.3
(22) Anmeldetag: 01.12.2006
(51) Int. Cl.: A61F 5/56

(54) **VORRICHTUNG ZUR SCHIENUNG EINES HOHLRAUMS, ORGANWEGS UND/ODER GEFÄSSES**
DEVICE FOR SPLINTING A CAVITY, ORGAN DUCT AND/OR VESSEL
DISPOSITIF POUR ASSURER LE MAINTIEN D'UNE CAVITE, D'UN CONDUIT ORGANIQUE ET/OU D'UN VAISSEAU

(30) Priorität: 06.12.2005 DE 102005058242; 29.08.2006 DE 102006040301
(43) Veröffentlichungstag der Anmeldung: 27.08.2008
(73) Patentinhaber: Düring, Klaus, Dr., 50226 Frechen (DE)
(72) Erfinder: MAHR, Richard, 88529 Schrobenhausen (DE); PFEFFER, Joachim, Georg, 52070 Aachen (DE)
(74) Vertreter: Ganahl, Bernhard
(86) Internationale Anmeldenummer: PCT/DE2006/002137
(87) Internationale Veröffentlichungsnummer: WO 2007/065408

(56) Entgegenhaltungen:
- WO-A-98/23233
- DE-A1- 10 240 725
- US-A1- 2001 044 647
- US-A1- 2003 153 973
- US-A1- 2004 134 491

## Beschreibung

Die vorliegende Erfindung betrifft eine Vorrichtung zur Schienung und/oder Offenhaltung eines Hohlraums, eines Organwegs und/oder eines Gefäßes im menschlichen oder tierischen Körper mit zumindest einem komprimierbaren und selbstexpandierenden Stent, der zumindest eine aufgeweitete Phase aufweist.

### Stand der Technik

In der US 2004/0134491 A1 geht eine Vorrichtung zur Behandlung von Schlafapnoe hervor. Diese Vorrichtung ist von der Größe her zum Platzieren in einer vorbestimmten Position in dem Oropharyngealen Bereich angepasst. Die Vorrichtung umfasst eine Schlaufe, die einen Innenraum begrenzt und einen ersten und einen zweiten Endbereich aufweist. Die Schlaufe ist durch zwei langgestreckte Elemente definiert die voneinander beabstandet angeordnet sind und sich zwischen dem ersten und den zweiten Endbereich erstrecken, wobei die Vorrichtung sich in einer C-förmigen auslandenden Bauform entfaltet, in welcher die langgestreckten Elemente gegen die Seitenwände des Oropharyngealen Bereichs drücken und auf diese Weise eine Öffnungskraft auf diesen Bereich ausüben.

In der US 2001/0044647 A1 ist ein modulares endoluminales Stent-Transplantat offenbart. Ein derartiges Stent-Implantat umfasst mindestens zwei Stent-Implantate unterschiedlicher Größe, wobei ein Stent-Implantat innerhalb des anderen entfaltet wird. Das erste Stent-Implantat weist ein chronisch aufgeweitetes Ende auf, dass zu einem Erstdurchmesser expandierbar ist, und einen mittleren Abschnitt der zu einem zweiten Durchmesser expandierbar ist, welcher kleiner als der Erstdurchmesser ist. Das zweite Stent-Implantat weist ein Ende auf das zu einem Durchmesser expandierbar ist, der in einen mittleren Abschnitt des ersten Stent-Implantats eingreift.

Aus der US 2003/0153973 A1 geht ein geflochtener modularer Stent mit Sanduhrförmigen Schnittstellen hervor. Der Stent umfasst eine erste Komponente und eine zweite Komponente, wobei jede Komponente eine stundenglasförmige Grenzfläche aufweist. Hierbei ist vorgesehen, dass ein Abschnitt mit verringertem Durchmesser eine größere radiale Festigkeit aufweist als Abschnitte mit nominellem Durchmesser, wobei der Abschnitt mit verringertem Durchmesser der stundenglasförmigen Grenzfläche ein größeren Flechtwinkel als die Abschnitte mit nominellem Durchmesser aufweist.

Krankheitsbilder, die auf dem zumindest partiellen Verschluß von Hohlräumen, Organwegen und/oder Gefäßen beruhen, nehmen zivilisationsbedingt stark zu. Ein wichtiger Bereich solcher Krankheitsbilder bezieht sich auf die Atemwege. So ist die obstruktive Schlafapnoe eine lebensbedrohliche Erkrankung, die aus dem Verschluß der hinteren Atemwege durch Muskelerschlaffung während des Schlafes resultiert. Durch wiederholten, teilweise sehr häufigen kurzzeitigen Atemstillstand wird der Patient während des Schlafs nicht ausreichend mit Sauerstoff versorgt, im Extremfall bis hin zur Todesfolge. Zivilisationsbedingte Faktoren wie z.B. starke Fettleibigkeit oder übermäßiger Alkoholgenuß verstärken das Erkrankungsrisiko deutlich. Das Schnarchen ist eine leichtere Effektausprägung als die Schlafapnoe, resultierend aus den gleichen organischen Ursachen. Während das Schnarchen dem Menschen offensichtlich ist, werden die schwerwiegenden gesundheitsgefährdenden Folgen vor allem des starken Schnarchens und der Schlafapnoe oft nicht registriert, da der Patient sich der Folgen der organischen Mangelerscheinungen nicht bewußt wird.

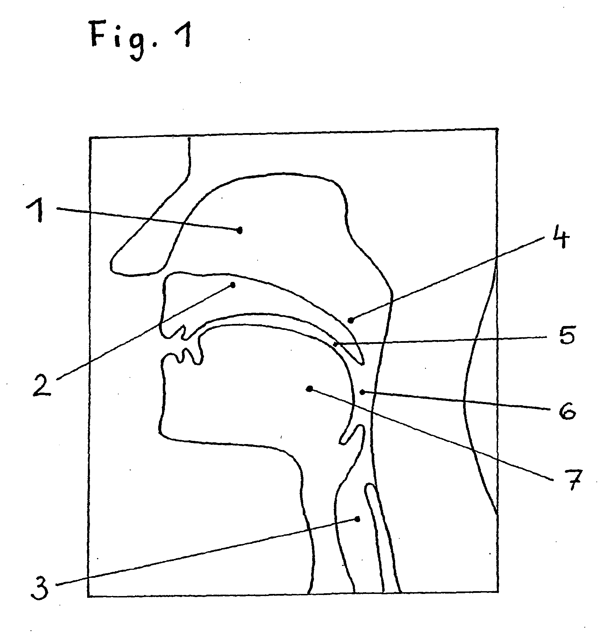

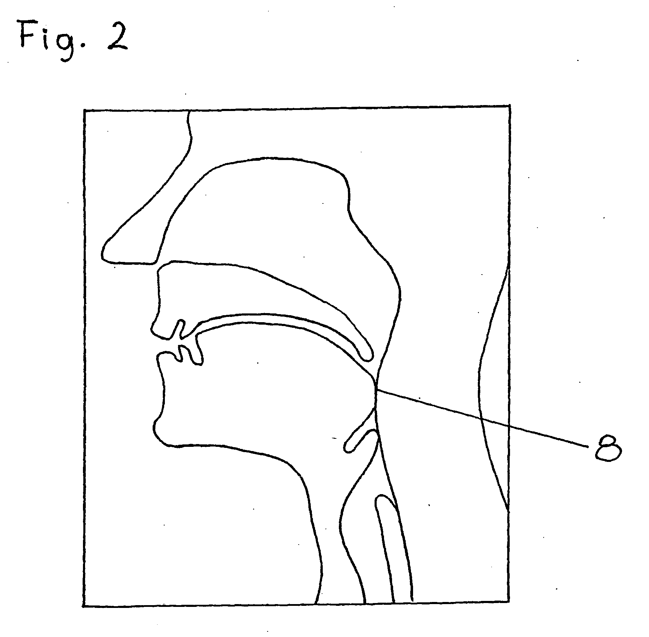

Fig. 1 zeigt freie Atemwege, bei denen eine normale Atmung gegeben ist. Die beim Schlafen eintretende Entspannung der Zunge und der umgebenden Muskeln im Rachenraum führt durch die hereinströmende Atemluft zu Schwingungen des Gaumensegels und der Weichteile des Rachens, was zum Schnarchgeräusch führt. Starkes Erschlaffen des Rachengewebes und das Zurückfallen des Zungengrundes kann zum völligen Verschluß der Atemwege führen (Fig. 2). Nach einer gewissen Zeit (ca. 60 - 90 Sekunden) wird durch das Gehirn eine Weckreaktion hervorgerufen. Der Übergang vom Schnarchen zur Apnoe ist fließend. Der Atemstillstand kann bis zu 1,5 Minuten andauern und sich 200-400 Mal während der Nacht wiederholen, was für den Patienten keinen Tiefschlaf zuläßt.

Die Folgen sind unruhiger Schlaf, Tagesmüdigkeit, Bluthochdruck, Herzbeschwerden, Antriebslosigkeit, usw. Wird die Krankheit nicht behandelt, so kann sich der Körper im Schlaf nicht ausreichend regenerieren, was zu einer geringeren Lebenserwartung führt. Verstärkte Druckschwankungen innerhalb des Brustkorbes können u.U. das Herz-Kreislaufsystem negativ beeinflußen. Folgen des Schnarchens sind Bluthochdruck und Abfall des Sauerstoffgehalts im Blut. Man nimmt heute an, daß insbesondere durch Schlafapnoe, aber auch durch Schnarchen wesentliche organische Folgeerkrankungen eintreten können.

Der Health Technology Assessment Report, Band 25 "Das Schlaf-Apnoe-Syndrom" von Perleth et al., Asgard Verlag St. Augustin 2003, konstatiert auf der Basis epidemiologischer Untersuchungen eine Prävalenz der Schlafapnoe von ca. 2-4% der Bevölkerung in Deutschland, also bei etwa 2 bis 3 Millionen betroffenen Menschen. Bei den in Schlaflabors durchgeführten diagnostischen Testungen erwiesen sich demnach etwa die Hälfte aller Patienten als von Apnoe betroffen und dringend behandlungsbedürftig. Andere Quellen gehen von einer Quote von nur 5% für die diagnostizierten und therapierten Erkrankungen aus. Nach Angaben von Prof. Dr. Frank Michael Baer, Universitätsklinikum Köln, gehört ein Drittel der männlichen Bevölkerung in Deutschland zu den regelmäßigen Schnarchern. Bei 4% der Männer und 2% der Frauen zwischen 30 und 60 Jahren müßte das Schnarchen behandelt werden. Dabei ist die Inzidenz des Schnarchens mit dem Alter stark ansteigend. So schnarchen im 20. Lebensjahr nur etwa 10% der Bevölkerung, im 50. Lebensjahr aber bereits etwa 50%. Zusammenfassend handelt es sich bei der Schnarchapnoe um eine Erkrankung von volkswirtschaftlich relevantem Charakter mit zunehmender Verbreitung und Bedeutung, wobei nur ein geringer Teil der Betroffenen erkannt ist und therapiert wird. Demnach besteht insbesondere auch ein Bedarf für neue, einfache, aber wirksame Therapiemöglichkeiten.

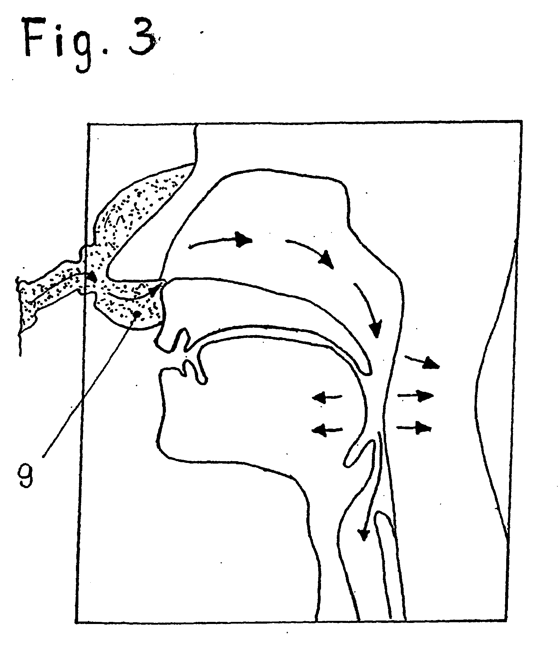

Die Standardtherapie bei Schlafapnoe ist heute die aktive Versorgung des Patienten mit Atemluft unter Überdruck durch ein n-CPAP-Beatmungsgerät (nasal continuous positive airway pressure). Durch das nächtliche Tragen einer Atemmaske, durch die ein kontinuierlicher positiver Atemwegsdruck erzeugt wird, werden die Atemwege ausreichend offengehalten. Das Wirkungsprinzip ist in Fig. 3 dargestellt. Wesentliche Nachteile dieser Standardtherapie, die bisher die einzige Behandlungsmöglichkeit mit medizinischer Effizienz ist, sind die Unannehmlichkeiten für den Patienten, die vor allem durch das luftdichte Tragen der Sauerstoffmaske während der ganzen Nacht, fixiert über ein ausreichend festes Kopfband und über den Versorgungsschlauch angebunden an das Beatmungsgerät, durch eine Austrocknung der Schleimhäute bzw. die Notwendigkeit der Befeuchtung der Atemluft sowie durch die Betriebsgeräusche des Beatmungsgeräts hervorgerufen werden. Zusätzlich zu der stark eingeschränkten Bewegungsfreiheit während des Schlafs kommt das Risiko, daß durch Undichtigkeiten zwischen der Atemmaske und der Gesichtshaut Luft entweicht und somit der Überdruck unbeabsichtigt reduziert wird, was zu einem nicht mehr ausreichend hohen Überdruck führen kann. Außerdem ist verständlicherweise die Akzeptanz für das nächtliche Tragen der Atemmaske bei vielen Patienten zeitlich sehr begrenzt, obwohl eine lebenslange Therapie erforderlich ist. Der o.g. HTA-Report berichtet, daß die Akzeptanz der CPAP-Behandlung insbesondere langfristig relativ gering ist. Weniger als die Hälfte der Patienten, denen CPAP empfohlen wurde, führen die Behandlung längerfristig (d.h. mehr als einen Monat) fort. Bemerkenswerterweise wird trotz nachweislich nur bei der CPAP-Maske gegebener signifikanter therapeutischer Wirksamkeit von den Patienten subjektiv eine intraorale Schiene eindeutig präferiert, was auf die geringere subjektive Belastung durch die Therapie zurückzuführen sein dürfte. Die intraorale Schiene soll zumindest eine teilweise Verbesserung der Symptome durch die Sicherstellung der Mundatmung bewirken. Ein weiterer Nachteil der CPAP-Maske ist die Notwendigkeit, das Beatmungsgerät auch auf Reisen mitführen zu müssen. Dazu kommt der Reinigungs- und Wartungsaufwand.

Aufgrund dieser starken Belastungen und Einschränkungen für den Patienten besteht ein hoher Leidensdruck unter den Apnoe-Patienten und ein starkes Interesse an alternativen und subjektiv verträglicheren Therapiemöglichkeiten. Nach vorherrschender Meinung ist bis heute keine effiziente alternative Behandlungsmöglichkeit entwickelt worden, die routinemäßig einsetzbar, praktikabel und kosteneffizient ist.

Eine Vielzahl von Vorschlägen zur mechanischen Verhinderung des Schnarchens und der Apnoe sind gemacht worden, von denen sich aber keiner auf dem Markt etablieren konnte. In DE 195 01 363 wird eine durch den Mund einzuführende Vorrichtung beschrieben, die durch ein in den Schlund einführbares und dort fixierbares Rohr gekennzeichnet ist und das Zusammenfallen der Weichteile im Schlund verhindern soll. Als ein Nachteil wird das notwendige Überwinden des Brechreizes vom Erfinder aufgeführt. Der hier beschriebene Weg über den Mund ist erfahrungsgemäß nicht praktikabel und führt zu keiner ausreichenden Minderung der Symptome. Eine ähnliche Vorrichtung zur Stützung der weichen Gaumenteile im Nasen- und Rachenbereich, die allerdings durch die Nase eingeführt werden soll, wird in DE 100 28 013 vorgeschlagen. Die Wirkung ist vergleichbar gering der des vorgenannten Instruments. Alternativ wird in WO 98/23233 vorgeschlagen, durch die Nase ein Doppelrohr zur Sicherstellung des Belüftungskanals einzuführen, das allerdings ähnlich wenig wirksam ist wie die vorgenannten beiden Instrumente. DE 102 40 725 beschreibt eine am Ende mittels eines Zugelementes aufweitbare Sonde gegen Apnoe und Schnarchen. Hierbei ist nicht beschrieben, wie in der Praxis das Aufweiten mittels des Zugelementes und die Stabilisierung der aufgeweiteten Sonde im Atemweg erfolgen soll.

### Aufgabenstellung

Der vorliegenden Erfindung liegt nun die Aufgabe zugrunde, einerseits die vorstehend genannten Nachteile des Standes der Technik zu vermeiden und andererseits eine praktikable Schienung und/oder Offenhaltung von Hohlräumen, Organwegen und/oder Gefäßen im menschlichen oder tierischen Körper zu ermöglichen, wobei für die Behandlung von Krankheiten wie Schnarchen und/oder Schlafapnoe eine täglich wiederholte Benutzung einer erfindungsgemäßen Vorrichtung notwendig ist und dadurch besondere Anforderungen an die Haltbarkeit eines derartigen medizinischen Instruments zu stellen sind. Dabei soll das erfindungsgemäße Prinzip für die Vorrichtung nicht nur spezifisch in den Atemwegen zur Behandlung von Schnarchen und/oder Schlafapnoe wirksam sein, sondern auch zur Schienung und/oder Offenhaltung anderer Hohlräume, Organwege und/oder Gefäße im menschlichen oder tierischen Körper angewendet werden können.

Die Aufgabe wird durch einen Stent mit den Merkmalen des Anspruchs 1 gelöst.

Dadurch wird eine Vorrichtung geschaffen, die im komprimierten Zustand in den Hohlraum, den Organweg und/oder das Gefäß eingeführt wird, wonach durch Zurückziehen eines äußeren Schlauches der innere zumindest eine Stent freigesetzt wird, sich selbständig aufweitet und sich zumindest mit der am stärksten aufgeweiteten Phase an die Wand des Hohlraums, Organwegs und/oder Gefäßes anlegt. Durch erneutes Aufschieben des Schlauches über den aufgeweiteten Stent kann die Vorrichtung wieder komprimiert und aus dem Hohlraum, Organweg und/oder Gefäß leicht entfernt werden. Durch den aufgeweiteten Stent wird der Hohlraum, der Organweg und/oder das Gefäß geschient und/oder offengehalten. Dabei ist der Schlauch mit dem beinhalteten komprimierten Stent so bemessen, daß er leicht in den Hohlraum, Organweg und/oder das Gefäß eingeführt werden kann, auch im kollabierten Zustand des Hohlraums, Organwegs und/oder Gefäßes. Vorzugsweise besteht der Stent aus einem Formgedächtnismaterial. Die Vorrichtung ist im Arbeitszustand vorzugsweise nur zu einem kleineren Teil in einer Phase des Stents aufgeweitet, wiederum bevorzugt am distalen Ende, wobei der andere Teil am anderen Ende des Stents nur eine geringere Aufweitung durch die Freisetzung aus dem Schlauch erfährt. Die Größen- und Längenverhältnisse der Phasen des zumindest einen Stents zueinander sind frei wählbar.

Durch die elastische Konstruktion des Stents und die Elastizität des umgebenden Schlauches kann die Vorrichtung leicht und effektiv einer Krümmung des Hohlraums, Organwegs und/oder Gefäßes folgen. Eine optimale Anpassung des Stents an die Anatomie des Hohlraums, Organwegs und/oder Gefäßes ist durch die variabel gestaltbare elastische Aufweitung des Stents sehr einfach möglich.

Vorzugsweise weist der Stent eine Netzstruktur und/oder eine Vielzahl von Öffnungen mit unterschiedlichen Öffnungswinkeln und/oder Öffnungsweiten in den Phasen auf. Besonders bevorzugt besteht der Stent aus einem Geflecht und/oder Gewebe und/oder Gelege, insbesondere aus einem Draht-, Faden- und/oder Fasergeflecht bzw. -gewebe bzw. -gelege. In einer anderen Ausführungsform kann der zumindest eine Stent aus einem zumindest über einen Teil seiner Länge geschlitzten Rohr bestehen, besonders bevorzugt hergestellt z.B. durch Laserschneiden eines Metall- oder Nitinolrohres.

Durch die aufgeweitete Phase mit dem größeren Durchmesser des zumindest einen Stents ist eine sichere und definierte Aufweitung zur Schienung und/oder Offenhaltung des Hohlraums, Organwegs und/oder Gefäßes nur in einem bestimmten, vordefinierten Bereich des Stents gewährleistet. In der mindestens einen proximalen Phase weist der Stent vorzugsweise kleinere Öffnungswinkel.in der Längsrichtung des Stents auf, in der mindestens einen distalen Phase vorzugsweise größere Öffnungswinkel in der Längsrichtung des Stents, damit die erforderliche Kraft zur Aufweitung, Schienung und/oder Offenhaltung des Hohlraums, Organwegs und/oder Gefäßes in diesem Bereich erreicht werden kann. In einer anderen bevorzugten Ausführungsform können die Öffnungswinkel in der Längsrichtung des Stents in der mindestens einen proximalen Phase größer und in der mindestens einen distalen Phase kleiner sein. In einer bevorzugten Ausführungsform mit mindestens drei Phasen besteht der Stent zur Schaffung eines Übergangsbereichs zwischen zwei funktionalen Phasen des Stents in der mindestens einen Übergangsphase vorzugsweise aus Öffnungen, die größer sind als diejenigen in den funktionalen Phasen, damit diese voneinander unbeeinflußt ihre Konfiguration nach der Freisetzung aus dem Schlauch annehmen können. Besonders bevorzugt wird dies erreicht durch eine Verzwirbelung von mindestens zwei Drähten, -fasern und/oder -fäden. In einer anderen bevorzugten Ausführungsform können diese Öffnungen durch Laserschneiden in einem Metall- oder Nitinolrohr erzeugt werden.

Das Material des zumindest einen Stents kann mit einer Beschichtung versehen werden, insbesondere mit einer biokompatiblen Oberflächenbeschichtung, Heparin, einer Carbonisierung von Nitinol, einer nanotechnologischen Beschichtung, röntgendichten Partikeln, einer einen Wirkstoff freisetzenden Beschichtung, einer insbesondere mikroporösen biotechnologischen oder einer anderen Beschichtung. Durch Vorsehen einer solchen Beschichtung kann zusätzlich zu der mechanischen Wirkung z.B. eine pharmazeutische Wirkung bezweckt werden. Bei Vorsehen einer die Oberfläche des Stents aufrauhenden Beschichtung ist außerdem eine bessere Fixierung des Stents am Wirkort möglich. Hierbei wird jedoch vorzugsweise darauf geachtet, daß eine Beschädigung der jeweiligen Wandung des Hohlraumes, Organwegs und/oder Gefäßes im menschlichen oder tierischen Körper, in dem der Stent angewendet wird, vermieden wird.

Vorzugsweise besteht der zumindest eine Stent aus einem biokompatiblen Formgedächtnismaterial, insbesondere einem Metall oder einer Metallegierung, insbesondere einem Edelstahl oder Nitinol oder einem anderen biokompatiblen Material, wie insbesondere einem Kunststoff oder monofilen und/oder multifilen und/oder koniposlten Glasfasern. Da der Stent zumindest einige Stunden bis Tage im menschlichen Körper verbleibt, erweist sich die Verwendung eines biokompatiblen Materials zum Vermeiden von Abstoßungseffekten und allergischen Reaktionen als besonders vorteilhaft. Um dem Stent die aufgeweitete Form einzuprägen, ihn nachfolgend zu komprimieren und sich selbstexpandierend öffnen zu lassen, wird vorteilhaft ein Formgedächtnismaterial gewählt.

In einer noch bevorzugteren Ausführungsform weist der Stent am distalen Ende runde Enden auf, d.h. der Draht bzw. die Faser bzw. der Faden ist in das Geflecht bzw. Gewebe bzw. Gelege zurückgeführt, ohne daß offene Draht- bzw. Faser- bzw. Fadenenden am distalen Ende des Stents entstehen, die zu einer Verletzung der jeweiligen Wandung des Hohlraumes, Organweges und/oder Gefäßes führen könnten. In einer noch mehr bevorzugten Ausführungsform sind die runden Enden am distalen Ende leicht nach innen gebogen.

In einer bevorzugten Ausführungsform wird das proximale Ende des Stents mit zumindest einem konstruktionsbedingten offenen Ende des Drahts bzw. der Faser bzw. des Fadens in einem Verschluß fixiert, z.B. geklebt, mit dem auch die Verbindung zu einem Zugelement hergestellt werden kann, mit dessen Hilfe der Stent mitsamt dem Schlauch in den Hohlraum, Organweg und/oder das Gefäß eingeführt und wieder aus diesem entfernt werden kann. In einer noch bevorzugteren Ausführungsform ist dieser Verschluß ein Gewindeverschluß.

Zur Sicherung des Stents gegen Hineinrutschen in den Hohlraum, Organweg und/oder das Gefäß kann der Stent am proximalen Ende mit einer Verriegelung ausgestattet werden, die in einer bevorzugten Ausführungsform variabel auf die jeweiligen anatomischen Gegebenheiten eingestellt werden kann. Der Schlauch kann länger, gleich lang oder kürzer sein als der Stent, ggf. noch verlängert um ein für die Handhabung notwendiges zusätzliches Stück abwärts des Stents, z.B. über das Zugelement hinweg, und kann damit den jeweiligen Einsatzbedingungen flexibel angepaßt werden. Die Vorrichtung kann in einer bevorzugten Anwendungsweise direkt bis zum Anwendungsort vorgeschoben und dort durch Zurückziehen des Schlauches der Stent freigesetzt werden. Der Schlauch kann dabei teilweise oder ganz zurückgezogen werden. In einer anderen bevorzugten Ausführungsform kann die Vorrichtung auch nur bis kurz vor den Anwendungsort vorgeschoben und dann der Stent aus dem Schlauch heraus an den Anwendungsort geschoben werden, wonach dann der Schlauch ganz oder teilweise zurückgezogen oder auch gar nicht zurückgezogen wird.

Für die Behandlung von Schnarchen und/oder Apnoe wird die Vorrichtung vorzugsweise so dimensioniert, daß das distale Ende des zumindest einen Stents gerade den Kehldeckel erreicht, um eine Reizung des Schlunds und Brechreiz zu vermeiden. Dabei wird die distale aufgeweitete Phase des Stents vorzugsweise in einer Länge von 0,5 cm bis 20 cm ausgebildet. Der gesamte Stent hat vorzugsweise eine Länge von 2 cm bis 50 cm. Der Schlauch hat vorzugsweise eine Länge von 0,5 cm bis 50 cm. In einer am meisten bevorzugten Ausführungsform hat der gesamte Stent eine Länge von 10 cm bis 35 cm mit einer distalen aufgeweiteten Phase zwischen 1 cm und 10 cm Länge und mit einem 10 cm bis 35 cm langen Schlauch. Es zeigt sich, daß mit einer derartigen Vorrichtung in den beschriebenen Bemaßungen bei der Anwendung beim Menschen zuverlässig und reproduzierbar die Atemwege während des Schlafes offengehalten werden können und die Sauerstoffsättigung im Blut bei einem Apnoeiker über den kritischen Grenzwert von 90% hinaus ansteigen kann. Somit ist ein Ersatz der n-CPAP-Atemmaske durch die erfindungsgemäße Vorrichtung zur Schienung und/oder Offenhaltung eines Hohlraums, Organwegs und/oder Gefäßes effizient, einfach und sehr gut körperverträglich möglich.

Analog zur Behandlung des Schnarchens und/oder der Schlafapnoe ist die Anwendung der erfindungsgemäßen Vorrichtung bespielsweise zur Behandlung von Tracheal- und Bronchialstenosen, Gallengangverengungen und zur Schienung und/oder Offenhaltung von Blutgefäßen, Lymphgefäßen, harnabführenden Wegen, Eileitern, Darmabschnitten möglich. Diese Aufzählung soll nicht limitierend sein für den Anwendungsbereich einer erfindungsgemäßen Vorrichtung, vielmehr nur die Breite des Anwendungsbereiches sichtbar machen.

### Ausführungsbeispiel

Zur näheren Erläuterung der Erfindung werden im folgenden Ausführungsbeispiele anhand der Zeichnungen näher beschrieben. In den Zeichnungen zeigen
Fig. 1 den freien Luftweg während des Schlafens,
Fig. 2 den blockierten Lufweg während des Schlafens als Ursache des Schnarchens und der obstruktiven Schlafapnoe, und
Fig. 3 die Öffnung der Atemwege durch n-CPAP-Therapie.
Fig. 4 zeigt eine Ansicht der Bauteile einer ersten Ausführungsform einer erfindungsgemäßen Vorrichtung zur Schienung und/oder Offenhaltung eines Hohlraums, Organwegs und/oder Gefäßes mit einem dreiphasigen Stent.
Fig. 5 zeigt eine Ansicht eines dreiphasigen Stents einer ersten Ausführungsform einer erfindungsgemäßen Vorrichtung.
Fig. 6 zeigt ein Flechtmuster mit runden Enden am distalen Ende des Geflechts bzw. Gewebes bzw. Geleges eines Stents einer zweiten Ausführungsform einer erfindungsgemäßen Vorrichtung, wobei immer nur ein Draht bzw. eine Faser bzw. ein Faden einzeln direkt zurückgeführt wird.
Fig. 7 zeigt ein Flechtmuster mit runden Enden am distalen Ende des Geflechts bzw. Gewebes bzw. Geleges eines Stents einer dritten Ausführungsform einer erfindungsgemäßen Vorrichtung, wobei sich immer zwei Drähte bzw. Fasern bzw. Fäden im Rückführungspunkt kreuzen.
Fig. 8 zeigt ein Flechtmuster mit runden Enden am distalen Ende des Geflechts bzw. Gewebes bzw. Geleges eines Stents einer dritten Ausführungsform einer erfindungsgemäßen Vorrichtung, wobei zumindest zwei Drähte bzw. Fasern bzw. Fäden zumindest teilweise parallel zurückgeführt werden.

Fig. 1 zeigt die Nasenhöhle (1), den harten Gaumen (2), die Luftröhre (3), den weichen Gaumen (4), die Mundhöhle (5), die freien Atemwege (6) sowie die Zunge (7). Dargestellt ist der freie Luftweg während des Schlafens beim gesunden Menschen. In Fig. 2 wird der verschlossene Luftweg (8) im Schlund, wie dies bei der obstruktiven Schlafapnoe eintritt, dargestellt. Fig. 3 zeigt die Anwendung des n-CPAP-Beatmungsgeräts mit der angelegten Atemmaske (9) und den durch den Überdruck geöffneten Atemwegen.

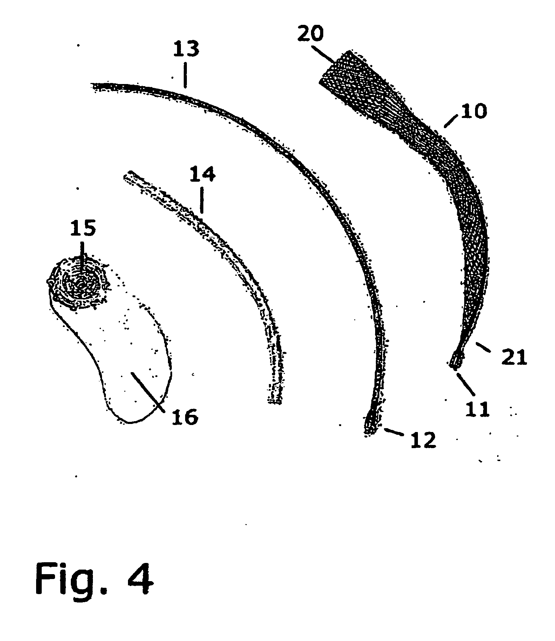

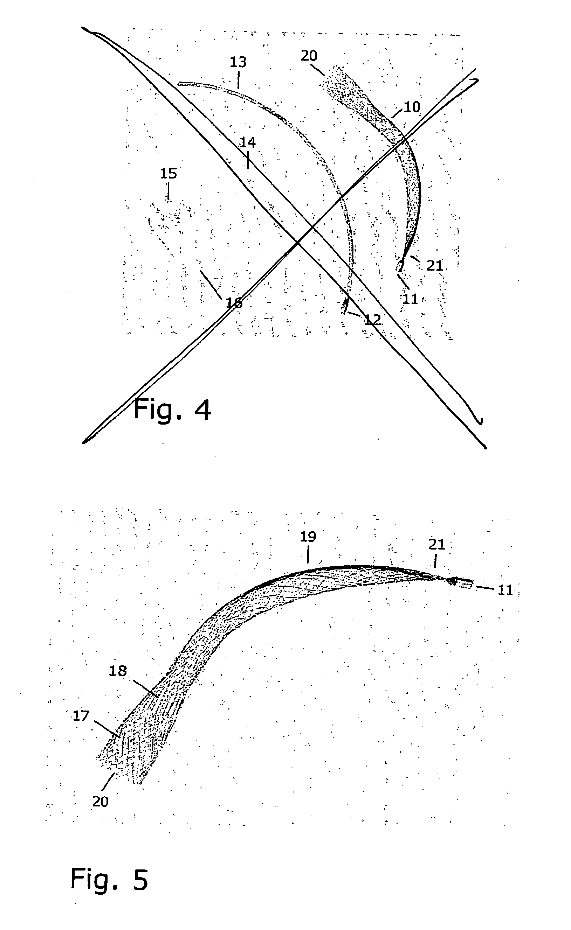

Fig. 4 zeigt eine Ansicht der Bauteile einer ersten Ausführungsform einer erfindungsgemäßen Vorrichtung zur Schienung und/oder Offenhaltung eines Hohlraums, Organwegs und/oder Gefäßes in einem menschlichen oder tierischen Körper. Die Vorrichtung umfaßt einen dreiphasigen Stent (10), der an seinem proximalen Ende (21) über ein Verbindungselement (11) mit dem Verbindungselement (12) eines Zugelementes (13) verbunden ist. Zum Einführen des Stents in den Hohlraum, Organweg und/oder das Gefäß wird der Stent mit Hilfe des Zugelementes in den Schlauch (14) eingeführt und komprimiert. An seinem proximalen Ende (21) wird der Stent (10) in einem Verbindungselement (11) fest fixiert. Hierdurch wird bei einem ggf. konstruktionsbedingten Vorhandensein von mindestens einem offenen Draht- bzw. Faser- bzw. Fadenende am proximalen Ende (21) des Stents (10) gleichzeitig dessen Zusammenhalt gewährleistet. Außerdem wird über das Verbindungselement (11), welches z.B. ein Schraubgewinde enthalten kann, die Verbindung zum Verbindungselement (12) des Zugelements (13), welches ebenfalls ein Schraubgewinde enthalten kann, hergestellt. Der Schlauch (14) kann vorzugsweise aus einem Kunststoffpolymer bestehen, beispielsweise ein PTFE-, PVC-, PE-, PU-, Silikon- oder Teflonschlauch, oder ein Katheter sein. Das Zugelement (13) kann bevorzugt aus einem Kunststoff, einem Metall oder einer Metallegierung oder Glasfaserwerkstoffen bestehen. In einer anderen Ausführungsform können die beiden Verbindungselemente (11) und (12) auch als ein einziges festes Element ausgebildet werden, so daß der Stent (10) fest mit dem Zugelement (13) verbunden ist.

Für die Anwendung einer erfindungsgemäßen Vorrichtung zur Behandlung von Schnarchen und/oder Schlafapnoe wird die Vorrichtung in im Schlauch (14) komprimiertem Zustand in eines der beiden Nasenlöcher eingeführt und vorzugsweise bis maximal zum Kehlkopfdeckel vorgeschoben und an ihrem Anwendungsort platziert. Durch Zurückziehen des Schlauches (14) wird der Stent (10) freigesetzt und expandiert selbständig in seine vorgeprägte Größe. Dadurch wird das Zusammenfallen des Schlundes und das Verschließen der Atemwege verhindert. Ein freier Luftstrom und eine normale Atmung wird dadurch ermöglicht. Zur Festlegung der Einführungstiefe und zur Sicherung gegen unbeabsichtigtes Hineinrutschen des Stents in den Atemweg kann in einer weiteren Ausführungsform eine Verriegelung (15) am proximalen Ende (21) des Stents (10) fixiert werden. Zur Sicherung gegen unbeabsichtigtes Hineinrutschen des Stents in die Atemwege und den Schlund kann außerdem eine Befestigungsplatte (16) montiert werden. Nach Einführung der Vorrichtung und Freisetzung des Stents (10) durch Zurückziehen des Schlauches (14) wird das Zugelement (13) mittels Lösen des Verbindungselements (12) vom Verbindungselement (11) vom Stent getrennt und entfernt. In dieser Platzierung und fixiert durch die Verriegelung (15) und ggf. die Befestigungsplatte (16) wird der Stent während des Schlafens getragen. In einer weiteren Ausführungsform kann noch eine weitere Befestigung des Stents am Kopf als Sicherung gegen Herausrutschen aus dem Atemweg erfolgen. Diese kann bespielsweise durch ein weiches Kopfband erfolgen, welches an der Befestigungsplatte befestigt werden kann.

In Fig. 5 ist eine Ansicht eines dreiphasigen Stents (10) in einer ersten Ausführungsform einer erfindungsgemäßen Vorrichtung zur Schienung und/oder Offenhaltung eines Hohlraums, Organwegs und/oder Gefäßes in einem menschlichen oder tierischen Körper für die Behandlung von Schnarchen und/oder Schlafapnoe dargestellt. Die distale funktionale Phase (17) des Stents ist in dieser ersten Ausführungsform der therapeutisch aktive Teil des medizinischen Instruments, der soweit aufgeweitet wird, daß er die Atemwege offenhält. Die proximale funktionale Phase (19) des Stents kann insbesondere der Fixierung des Stents im Nasenbereich dienen und wird dementsprechend nur geringer aufgeweitet. Die distale Phase (17) weist also einen größeren Aufweitungsdurchmesser auf als die proximale Phase (19). Um die unterschiedliche Aufweitung der distalen Phase (17) und der proximalen Phase (19) des Stents (10) sicher zu gewährleisten und dies auch für eine vielfach wiederholte Benutzung der Vorrichtung, die jeweils aus einem Öffnungs- und aus einem Schließungsvorgang besteht, zu erreichen, sind die distale Phase (17) und die proximale Phase (19) durch eine vorzugsweise verzwirbelte Übergangsphase (18) miteinander verbunden, die durch die Stabilität der verzwirbelten Drähte, Fasern bzw. Fäden den endständigen Phasen die voneinander unbeeinflußte Einnahme ihrer Konfiguration nach Freisetzung aus dem Schlauch ermöglicht. Die Ausführung des Stents (10) in dieser mindestens dreiphasigen Form aus mindestens zwei funktionalen Phasen und mindestens einer Übergangsphase ist ein wesentliches Element einer Ausführungsform einer erfindungsgemäßen Vorrichtung und unterscheidet den Stent (10) somit wesentlich von den heute allgemein üblichen Stents zur Kanalisierung oder Offenhaltung von z.B. Blutgefäßen.

Grundsätzlich ist es frei wählbar, welche Phase der mindestens drei Phasen des zumindest einen Stents (10) den größten Durchmesser im aufgeweiteten Zustand einnimmt. Dabei kann eine Übergangsphase (18) entweder den gleichen oder einen größeren oder auch einen kleineren Durchmesser aufweisen als die funktionalen Phasen. Die Übergangsphase (18) wird vorzugsweise durch Verzwirbelung von mindestens zwei Drähten bzw. Fasern bzw. Fäden konstruiert, während die anderen Phasen vorzugsweise durch Kreuzung von mindestens einem Faden konstruiert werden. Für die Konstruktion der weniger aufgeweiteten Phase wird vorzugsweise ein Geflecht, Gewebe und/oder Gelege mit kleineren Öffnungswinkeln in der Längsrichtung des Stents gewählt. Für die Konstruktion der stärker aufgeweiteten Phase wird vorzugsweise ein Geflecht, Gewebe und/oder Gelege mit größeren Öffnungswinkeln in Längsrichtung des Stents gewählt.

Um eine besonders gute Stabilität des Stents bei gleichzeitiger optimaler Luftdurchlässigkeit zu erreichen, weist dieser eine Netzstruktur auf. Diese kann z.B. durch ein Draht-, Faden- und/oder Fasergeflecht bzw. -gewebe bzw. -gelege gebildet werden. Alternativ kann auch ein geschnittenes Röhrchen mit einer solchen Struktur unterschiedlich großer Öffnungen versehen werden, besonders bevorzugt durch Laserschneiden eines Metall- oder Nitinolrohres. Wichtig sind dabei eine Anzahl von Öffnungen, um einen ungehinderten Luftstrom zu ermöglichen. Der Stent kann somit ein sehr feines Netzwerk aufweisen oder ein gröberes. In einer bevorzugten Ausführungsform wird der Stent aus einem Formgedächtsnismaterial hergestellt. Dies kann beispielsweise Nitinol, Edelstahl, Kunststoff oder monofiles, multifiles und/oder komposites Glasfasermaterial sein. In einer bevorzugten Ausführungsform wird der Stent durch manuelles oder maschinelles Flechten in vordefinierten Mustern zur Erzeugung der unterschiedlichen Phasen hergestellt.

Ein besonderes Problem beim wiederholten Einführen und Entfernen einer erfindungsgemäßen Vorrichtung einerseits und der möglichen Anwendung in offenen Hohlräumen andererseits liegt darin, daß bei offenen Enden eines Materials, aus dem das Geflecht bzw. Gewebe bzw. Gelege des Stents (10) hergestellt wird, möglicherweise eine Reizung der Wandung des Hohlraums, Organweges und/oder Gefäßes resultieren kann. Um dies zu vermeiden, sind geschlossene Enden des Stents (10) zumindest am distalen Ende (20) des Stents zu bevorzugen. Geschlossene Enden vor allem in geflochtenen Systemen sind heute kein allgemeingültiger Stand bei kommerziell erhältlichen Stents. Vielmehr enthalten diese üblicherweise beidseitig offene Enden. Für die erfindungsgemäße Vorrichtung wurden daher spezielle neuartige Flechtmuster entwickelt und erfindungsgemäße Stents hergestellt. Dabei kann der Stent (10) vorzugsweise aus nur einem Draht bzw. einer Faser bzw. einem Faden hergestellt werden, was am distalen Ende (20) des Stents (10) ausschließlich geschlossene Enden produziert und am proximalen Ende (21) des Stents (10) zwei offene Draht- bzw. Faser- bzw. Fadenenden. In einer anderen bevorzugten Ausführungsform werden soviele Drähte bzw. Fasern bzw. Fäden zur Herstellung des Stents (10) eingesetzt, wie geschlossene Enden erzeugt werden sollen. Dies können beispielsweise 12, 18 oder 24 Drähte bzw. Fasern bzw. Fäden für 12, 18 oder 24 geschlossene Enden am distalen Ende (20) des Stents (10) sein. Dadurch entstehen 24, 36 oder 48 offene Draht- bzw. Faser- bzw. Fadenenden am proximalen Ende (21) des Stents (10). Da das proximale Ende (21) des Stents (10) in dem Verbindungselement (11) fest fixiert wird, sind alle offenen Enden der Drähte bzw. Fasern bzw. Fäden am proximalen Ende (21) des Stents (10) in jedem Fall fest eingebunden und stellen keine Gefährdung des Patienten dar.

Bei der Auswahl der Stärke des Drahtes bzw. der Faser bzw. des Fadens sind die Materialeigenschaften ausgewogen abzustimmen mit den mechanischen Eigenschaften des Geflechts bzw. Gewebes bzw. Geleges. In einer bevorzugten Ausführungsform wird der Stent (10) aus Nitinoldraht mit einem Durchmesser von 0,001 mm bis 2 mm hergestellt, noch mehr bevorzugt mit einem Durchmesser von 0,05 mm bis 0,5 mm, und am meisten bevorzugt mit einem Durchmesser von 0,1 mm bis 0,2 mm. Der Durchmesser des Nitinoldrahtes, die Dichte und der Öffnungswinkel des Geflechts bestimmen die Kräfte, mit denen die stärker aufgeweitete Phase des Stents (10) den Hohlraum, Organweg und/oder das Gefäß aufweitet und offenhält. Diese Kräfte tragen bei bestimmten Behandlungsformen wie der von Schnarchen und/oder Schlafapnoe wesentlich zur Erreichung des Wohlbefindens des Patienten bei.

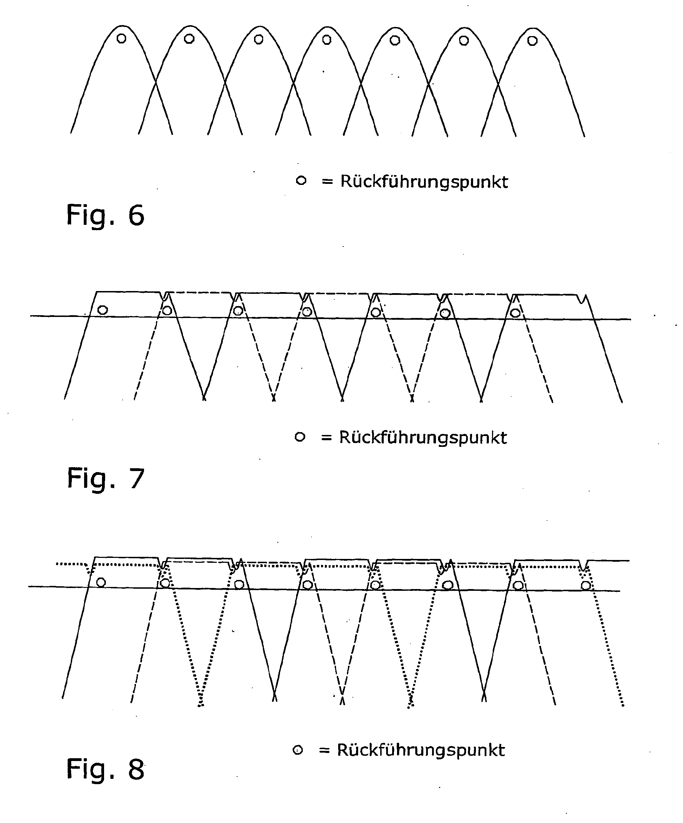

In Fig. 6 wird ein Flechtmuster mit runden Enden am distalen Ende (20) des Stents (10) in einer bevorzugten Ausführungsform einer erfindungsgemäßen Vorrichtung dargestellt, wobei immer nur ein Draht bzw. eine Faser bzw. ein Faden einzeln in das Geflecht zurückgeführt wird. In einer bevorzugten Ausführungsform wird der/die ankommende Draht bzw. Faser bzw. Faden direkt rund zurückgeführt in das Geflecht. In einer weiteren bevorzugten Ausführungsform (Fig. 7) wird der/die ankommende Draht bzw. Faser bzw. Faden so zurückgeführt, daß sich immer zwei Drähte bzw. Fasern bzw. Fäden im Rückführungspunkt kreuzen. Diese Ausführungsbeispiele sollen nicht limitierend sein für mögliche Flechtmuster zur Rückführung eines/einer ankommenden Drahtes bzw. Faser bzw. Fadens, vielmehr sind viele weitere entsprechende Ausführungsformen unter dem im Vorstehenden Beschriebenen und in den Fig. 6 und 7 Dargestellten möglich, um runde Enden am distalen Ende (20) des Stents (10) in einem Geflecht zu erhalten, wobei jeweils nur ein(e) einzelne(r) Draht bzw. Faser bzw. Faden zurückgeführt wird. Diese werden hier eingeschlossen.

In Fig. 8 wird ein Flechtmuster mit runden Enden am distalen Ende (20) des Stents (10) in einer bevorzugten Ausführungsform einer erfindungsgemäßen Vorrichtung dargestellt, wobei zumindest zwei Drähte bzw. Fasern bzw. Fäden zumindest teilweise parallel zurückgeführt werden. In einer bevorzugten Ausführungsform wird der/die ankommende Draht bzw. Faser bzw. Faden rund zurückgeführt in die zweite Position innerhalb des Flechtmusters. In weiteren bevorzugten Ausführungsformen wird der/die ankommende Draht bzw. Faser bzw. Faden rund zurückgeführt in die dritte oder danach folgende Positionen innerhalb des Flechtmusters. Diese Ausführungsbeispiele sollen nicht limitierend sein für mögliche Flechtmuster zur Rückführung eines/einer ankommenden Drahtes bzw. Faser bzw. Fadens, vielmehr sind viele weitere entsprechende Ausführungsformen unter dem im Vorstehenden Beschriebenen und in der Fig. 8 Dargestellten möglich, um runde Enden am distalen Ende (20) des Stents (10) in einem Geflecht zu erhalten, wobei zumindest zwei Drähte bzw. Fasern bzw. Fäden zumindest teilweise parallel zurückgeführt werden. Diese werden hier eingeschlossen. Der Vorteil der in Fig. 7 und 8 dargestellten und ähnlicher Flechtmuster liegt darin, daß einerseits das distale Ende (20) des Stents (10) eine höhere Stabilität erhält, andererseits ein größere Rundung gegenüber den in Fig. 6 dargestellten und ähnlichen Flechtmustern erzielt werden können. Letzteres trägt zum Anwendungskomfort für den Patienten bei.

In dem im Vorstehenden Beschriebenen und in den in den Figuren dargestellten Ausführungsformen einer Vorrichtung zur Schienung und/oder Offenhaltung eines Hohlraums, Organweges und/oder Gefäßes im menschlichen oder tierischen Körper können noch zahlreiche weitere Ausführungsformen gebildet werden. Insbesondere kann die Form des Stents auf die Form des Hohlraums bzw. Organwegs bzw. Gefäßes angepaßt werden. Teilweise eignen sich eher kürzere Stents mit einem größeren Öffnungsdurchmesser, bei anderen Anwendungsstellen können eher schmalere, längere Stents vorteilhaft sein. In den im Vorstehenden Beschriebenen und in den in den Figuren dargestellten Ausführungsformen eines mindestens dreiphasigen Stents können ebenfalls noch zahlreiche weitere Ausführungsformen gebildet werden. Bei jeder dieser Ausführungsformen ist mindestens eine funktionale Phase des Stents mit mindestens einer weiteren funktionalen Phase durch eine Übergangsphase, die die freie Ausbildung der beiden unterschiedlichen Konfigurationen der funktionalen Phasen befördert, verbunden.

### Bezugszeichenliste

- 1: Nasenhöhle
- 2: Harter Gaumen
- 3: Luftröhre
- 4: Weicher Gaumen
- 5: Mundhöhle
- 6: Freie Atemwege
- 7: Zunge
- 8: verschlossene Luftwege
- 9: Nasenmaske des n-CPAP-Gerätes
- 10: Stent
- 11: Verbindungselement
- 12: Verbindungselement
- 13: Zugelement
- 14: Schlauch
- 15: Verriegelung
- 16: Befestigungsplatte
- 17: distale funktionale Phase des Stents
- 18: Übergangsphase des Stents
- 19: proximale funktionale Phase des Stents
- 20: distales Ende des Stents
- 21: proximales Ende des Stents

## Patentansprüche

1. Komprimierbarer, selbstexpandierender Stent (10), wobei der Stent aus mindestens drei Phasen aufgebaut ist, wobei der Stent (10) aus zumindest einem Draht, einer Faser oder einem Faden ausgebildet ist, und eine distale funktionale Phase (17), eine proximale funktionale Phase (19) und eine Übergangsphase (18) vorgesehen sind und die distale Phase (17) eine größere Aufweitung als die proximale Phase (19) aufweist,
wobei die funktionalen Phasen (17, 19) aus einem Geflecht und/oder Gewebe und/oder Gelege bestehen,
wobei in der Übergangsphase (18) jeweils mindestens zwei Abschnitte des zumindest einen Drahtes bzw. der Drähte, der zumindest einen Faser bzw. der Fasern oder des zumindest einen Faden oder der Fäden miteinander verzwirbelt sind.

2. Stent nach Anspruch 1, **dadurch gekennzeichnet, dass** der Stent (10) eine Netzstruktur und/oder eine Vielzahl von Öffnungen aufweist, wobei die Öffnungen der Übergangsphase (18) größer sind als die der funktionalen Phasen (17, 19).

3. Stent nach Anspruch 1 oder 2, **dadurch gekennzeichnet, dass** der Draht, die Faser oder der Faden vorzugsweise einen Durchmesser von 0,001 mm bis 2 mm, insbesondere einen Durchmesser von 0,05 mm bis 0,5 mm und vorzugsweise einen Durchmesser von 0,1 mm bis 0,2 mm hat.

4. Stent nach einem der Ansprüche 1 bis 3, **dadurch gekennzeichnet, dass** der Stent aus einem Formgedächtnismaterial hergestellt ist.

5. Stent nach Anspruch 4, **dadurch gekennzeichnet, dass** das Formgedächtnismaterial ein biokompatibles Formgedächtnismaterial ist, insbesondere ein Metall oder eine Metalllegierung, insbesondere Edelstahl oder Nitinol oder ein anderes biokompatibles Material, wie Kunststoff oder monofile und/oder multifile und/oder komposite Glasfasern.

6. Stent nach einem der Ansprüche 1 bis 5, **dadurch gekennzeichnet, dass** das distale Ende (20) des Stents ausschließlich geschlossene Enden aufweist.

7. Stent nach einem der Ansprüche 1 bis 6, **dadurch gekennzeichnet, dass** das zumindest eine offene Ende am proximalen Ende (21) in einem Verbindungselement (11) fest fixiert ist.

8. Stent nach einem der Ansprüche 1 bis 7, **dadurch gekennzeichnet, dass** das distale Ende (20) 12, 18 oder 24 geschlossene Enden aufweist.

9. Stent nach Anspruch 8, **dadurch gekennzeichnet, dass** der Stent (10) aus soviel Drähten bzw. Fasern bzw. Fäden ausgebildet ist, wie geschlossene Enden vorgesehen sind.

10. Stent nach einem der Ansprüche 1 bis 8, **dadurch gekennzeichnet, dass** der Stent (10) aus nur einem Draht bzw. einer Faser bzw. einem Faden ausgebildet ist.

11. Stent nach einem der Ansprüche 1 bis 10, **dadurch gekennzeichnet, dass** das Material des Stents mit einer Beschichtung versehen ist, insbesondere einer biokompatiblen Oberflächenbeschichtung, Heparin, einer Carbonisierung von Nitinol, einer nanotechnologischen Beschichtung, röntgendichten Partikeln, einer einen Wirkstoff freisetzenden Beschichtung, einer insbesondere mikroporösen biotechnologischen oder einer anderen Beschichtung.

12. Vorrichtung zur Schienung und/oder Offenhaltung eines Hohlraums, Organwegs und/oder Gefäßes im menschlichen oder tierischen Körper mit zumindest einem in einem Schlauch (14) komprimierbaren und selbstexpandierenden Stent (10), wobei der Stent (10) ein Stent nach einem der Ansprüche 1 bis 11 ist.

13. Vorrichtung nach Anspruch 12, **dadurch gekennzeichnet, dass** die distale Phase (17) des Stents (10) eine Länge von 0,5 cm bis 20 cm, der Schlauch (14) eine Länge von 0,5 cm bis 50 cm und der gesamte Stent (10) eine Länge von 2 cm bis 50 cm aufweist.

14. Vorrichtung nach Anspruch 12 oder 13, **dadurch gekennzeichnet, dass** der zumindest eine Stent an seinem proximalen Ende über Verbindungselemente (11, 12) mit einem Zugelement (13) verbunden werden kann, mit dessen Hilfe der zumindest eine Stent in den Schlauch (14) eingezogen werden kann.

15. Vorrichtung nach einem der Ansprüche 12 bis 14, **dadurch gekennzeichnet, dass** die distale Phase (17) des Stents (10) eine Länge von 1 cm bis 10 cm, der Schlauch (14) eine Länge von 10 cm bis 35 cm und der gesamte Stent (10) eine Länge von 10 cm bis 35 cm aufweist.

## Claims

1. Compressible, self-expanding stent (10) **characterised in that** the stent is made up of at least three phases, wherein the stent (10) is made of at least one wire, one fibre or one thread, and a distal functional phase (17), a proximal functional phase (19) and a transition phase (18) are provided, and the distal phase (17) provides a greater degree of expansion than the proximal phase (19),
wherein the functional phases (17, 19) are made of a braiding and/or woven fabric and/or interlaid scrim,
wherein in the transition phase (18) at least two segments of the at least one wire or of the wires, resp., of the at least one fibre or the fibres, resp., or of the at least one thread or the threads, resp., are twisted together.

2. Stent according to claim 1, **characterised in that** the stent (10) has a net structure and/or a multiplicity of openings, wherein the openings of the transition phase (18) are larger than those of the functional phases (17, 19).

3. Stent according to claim 1 or 2, **characterised in that** the wire, the fibre or the thread preferably has a diameter of 0.001 mm to 2 mm, particularly a diameter of 0.05 mm to 0.5 mm, and preferably a diameter of 0.1 mm to 0.2 mm.

4. Stent according to any of claims 1 to 3, **characterised in that** the stent is made of a shape memory material.

5. Stent according to claim 4, **characterised in that** the shape memory material is a bio-compatible shape memory material, in particular a metal or a metal alloy, in particular stainless steel or nitinol or another bio-compatible material, such as a plastic, or monofile and/or multifile and/or composite glass fibres.

6. Stent according to any of claims 1 to 5, **characterised in that** the distal end (20) of the stent has exclusively closed ends.

7. Stent according to any of claims 1 to 6, **characterised in that** the at least one open end at the proximal end (21) is firmly fixed in a closing element (11).

8. Stent according to any of claims 1 to 7, **characterised in that** the distal end (20) has 12, 18 or 24 closed ends.

9. Stent according to claim 8, **characterised in that** the stent (10) is made of the same number of wires or fibres or threads, resp., as the number of closed ends is.

10. Stent according to any of the claims 1 to 8, **characterised in that** the stent (10) is made of only one wire or one fibre or one thread, resp.

11. Stent according to any of claims 1 to 10, **characterised in that** the material of the stent is provided with a coating, in particular a bio-compatible surface coating, heparin, a carbonisation of nitinol, a nano-technological coating, radiopaque particles, a coating releasing an active substance, a preferably microporous biotechnological, or another coating.

12. Device for the splinting and/or holding open of a cavity, organ duct and/or vessel in the human or animal body with at least one stent (10) which is compressible in a tube (14) and is self-expanding, wherein the stent (10) is a stent according to any of claims 1 to 11.

13. Device according to claim 12, **characterised in that** the distal phase (17) of the stent (10) has a length of 0.5 cm to 20 cm, the tube (14) a length of 0.5 cm to 50 cm and the whole stent (10) a length of 2 cm to 50 cm.

14. Device according to any of claims 12 or 13, **characterised in that** the at least one stent may be connected at its proximal end via connecting elements (11, 12) to a pulling element (13) which may be used to draw the at least one stent into the tube (14).

15. Device according to any of claims 12 to 14, **characterised in that** the distal phase (17) of the stent (10) has a length of 1 cm to 10 cm, the tube (14) a length of 10 cm to 35 cm and the whole stent (10) a length of 10 cm to 35 cm.

## Revendications

1. Stent (10) auto-expansible compressible, dans lequel le stent est conçu en au moins trois phases, dans lequel le stent (10) est réalisé à partir d'au moins un fil, une fibre ou un filament, et une phase fonctionnelle distale (17), une phase fonctionnelle proximale (19) et une phase de transition (18) sont prévues et la phase distale (17) présente un plus grand élargissement que la phase proximale (19),
dans lequel les phases fonctionnelles (17, 19) consistent en un tressage et/ou un tissu et/ou un non-tissé,
dans lequel, dans la phase de transition (18), au moins deux segments de l'au moins un fil ou des fils, de l'au moins une fibre ou des fibres, ou de l'au moins un filament ou des filaments sont entortillés l'un avec l'autre.

2. Stent selon la revendication 1, **caractérisé par le fait que** le stent (10) présente une structure de filet et/ou une pluralité d'ouvertures, les ouvertures de la phase de transition (18) étant plus grandes que celles des phases fonctionnelles (17, 19).

3. Stent selon la revendication 1 ou 2, **caractérisé par le fait que** le fil, la fibre ou le filament a un diamètre de 0,001 mm à 2 mm, en particulier un diamètre de 0,05 mm à 0,5 mm et plus préférablement un diamètre de 0,1 mm à 0,2 mm.

4. Stent selon l'une des revendications 1 à 3, **caractérisé par le fait que** le stent est réalisé en un matériau à mémoire de forme.

5. Stent selon la revendication 4, **caractérisé par le fait que** le matériau à mémoire de forme est un matériau à mémoire de forme biocompatible, en particulier un métal ou un alliage de métal, en particulier de l'acier inoxydable ou du nitinol ou un autre matériau biocompatible, tel que le plastique ou les fibres de verre monofil et/ou multifil et/ou composites.

6. Stent selon l'une des revendications 1 à 5, **caractérisé par le fait que** l'extrémité distale (20) du stent présente exclusivement des extrémités fermées.

7. Stent selon l'une des revendications 1 à 6, **caractérisé par le fait que** l'au moins une extrémité ouverte à l'extrémité proximale (21) est fixée fermement dans un élément de connexion (11).

8. Stent selon l'une des revendications 1 à 7, **caractérisé par le fait que** l'extrémité distale (20) présente 12, 18 ou 24 extrémités fermées.

9. Stent selon la revendication 8, **caractérisé par le fait que** le stent (10) est réalisé en autant de fils ou de fibres ou de filaments qu'il est prévu d'extrémités fermées.

10. Stent selon l'une des revendications 1 à 8, **caractérisé par le fait que** le stent (10) est réalisé en un seul fil ou une seule fibre ou un seul filament.

11. Stent selon l'une des revendications 1 à 10, **caractérisé par le fait que** le matériau du stent est muni d'un revêtement, en particulier un revêtement superficiel biocompatible, héparine, une carbonisation de nitinol, un revêtement nanotechnologique, des particules étanches aux rayons X, un revêtement dégageant une substance, un revêtement en particulier micro-poreaux biotechnologique ou autre.

12. Dispositif pour éclisser et/ou maintenir ouverte une cavité, une voie d'organe et/ou un vaisseau dans le corps humain ou animal avec au moins un stent (10) compressible et auto-expansible dans un flexible (14), le stent (10) étant un stent selon l'une des revendications 1 à 11.

13. Dispositif selon la revendication 12, **caractérisé par le fait que** la phase distale (17) du stent (10) présente une longueur de 0,5 cm à 20 cm, le flexible présente une longueur de 0,5 cm à 50 cm et l'ensemble du stent (10) présente une longueur de 2 cm à 50 cm.

14. Dispositif selon la revendication 12 ou 13, **caractérisé par le fait que** l'au moins un stent peut être connecté, à son extrémité proximale, par l'intermédiaire d'éléments de connexion (11, 12), à un élément de traction (13) à l'aide duquel l'au moins un stent peut être tiré dans le flexible (14).

15. Dispositif selon l'une des revendications 12 à 14, **caractérisé par le fait que** la phase distale (17) du stent (10) présente une longueur de 1 cm à 10 cm, le flexible (14) présente une longueur de 10 cm à 35 cm et l'ensemble du stent (10) présente une longueur de 10 cm à 35 cm.
